(19) Europäisches Patentamt
European Patent Office
Office européen des brevets

(11) Numéro de publication : **0 538 080 A1**

# (12) DEMANDE DE BREVET EUROPEEN

(21) Numéro de dépôt : **92402469.8**

(22) Date de dépôt : **10.09.92**

(51) Int. Cl.$^5$ : **C07D 211/26,** C07D 405/12, C07D 409/12, C07D 411/12, A61K 31/445

(30) Priorité : **16.09.91 FR 9111382**

(43) Date de publication de la demande : **21.04.93 Bulletin 93/16**

(84) Etats contractants désignés : **AT BE CH DE DK ES FR GB GR IT LI LU MC NL PT SE**

(71) Demandeur : **PIERRE FABRE MEDICAMENT
45, Place Abel Gance
F-92100 Boulogne (FR)**

(72) Inventeur : **Bonnaud, Bernard
75, Route de Pioch
F-81090 Lagarrigue (FR)**
Inventeur : **Bigg, Dennis
122, avenue de Lavaur
F-81100 Castres (FR)**

(74) Mandataire : **Doat, Jean Pierre
17, Avenue Jean Moulin
F-81106 Castres Cedex (FR)**

## (54) Dérivés de l'aminométhyl-4 pipéridine, leur préparation et leur application en thérapeutique.

(57) 1) Dérivés de l'aminométhyl-4 pipéridine correspondant à la formule générale 1

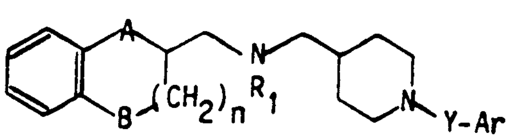

1

ainsi que les sels thérapeutiquement acceptables de ces molécules.

L'invention concerne également l'application des composés de formule générale 1 en thérapeutique et les procédés de préparation.

EP 0 538 080 A1

La présente invention, réalisée au Centre de Recherche Pierre Fabre, a pour objet de nouveaux composés chimiques, leur préparation et leur application en thérapeutique.

Les composés de l'invention répondent à la formule générale 1 :

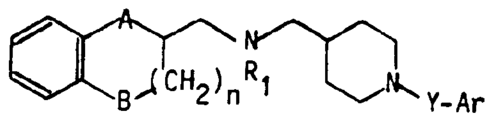

1

dans laquelle :

A et B représentent, indépendamment l'un de l'autre, un atome d'oxygène, un atome de soufre, ou un radical méthylène;

n peut prendre les valeurs de 0 ou 1 ;

$R_1$ représente un atome d'hydrogène ou un groupe alkyle en $C_1$-$C_4$ ;

Y représente un radical carbonyle ou un radical méthylène ;

Ar représente un groupe phényle substitué ou non par un ou plusieurs substituants choisis parmi un atome d'halogéne, un groupe alkyle en $C_1$-$C_4$, un groupe alkoxy en $C_1$-$C_4$ ou un groupe trifluorométhyle.

L'invention concerne également les sels des composée de formule générale 1 avec des acides minéraux ou organiques pharmaceutiquement acceptables. L'acide employé peut être, à titre d'exemple non limitatif, l'acide chlorhydrique, l'acide fumarique ou l'acide maléique.

Quand les composés de formule générale 1 renferment un carbone asymétrique, la présente invention concerne aussi bien les mélanges racémiques que les différents énantiomères ou leurs mélanges en toutes proportions.

Les composés de formule générale 1, où Y représente un radical carbonyle, peuvent être préparés selon le schéma de réaction :

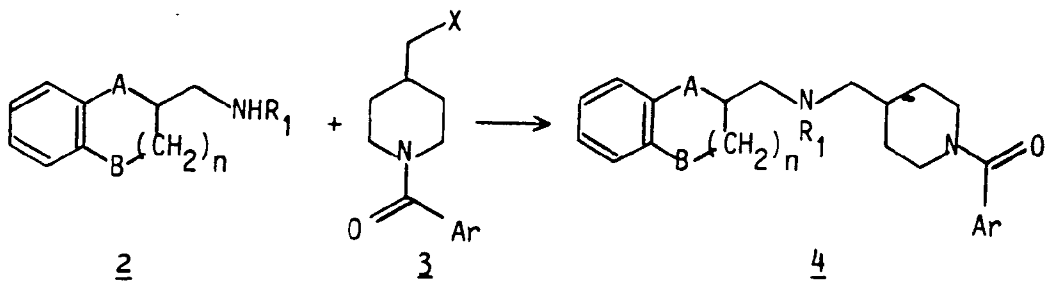

2 3 4

où

. A, B, n, $R_1$ et Ar sont définis comme ci-dessus.

. X représente un groupe nucléofuge tel que méthylsulfonyloxy, benzènesulfonyloxy ou p-toluènesulfonyloxy.

Les amines de départ 2 et les pipéridines de formule 3 peuvent être obtenues selon des méthodes classiques.

La réaction entre un composé de formule générale 2 et un composé de formule générale 3 s'effectue, soit en l'absence, soit en présence d'un solvant tel que le toluène, le xylène, le diméthylformamide ou l'acétonitrile de préférence à une température comprise entre 50 et 160°C, et éventuellement, en présence d'une base organique telle qu'une amine tertiaire, ou minérale, telle qu'un carbonate ou hydrogénocarbonate alcalin. On obtient ainsi un composé de formule générale 4 qui correspond à la formule générale 1 lorsque Y représente le radical carbonyle.

Les composés de formule générale 1, où Y représente un radical méthylène, peuvent être préparés selon le schéma de réaction :

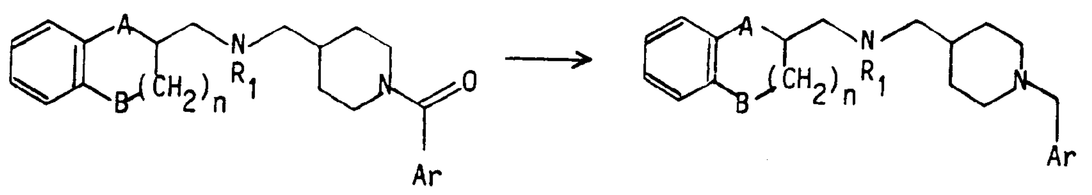

4

où A, B, n, $R_1$ et Ar sont définis comme ci-dessus.

La réduction d'un composé de formule générale 4, obtenue selon les méthodes décrites ci-dessus, s'effectue au moyen d'un hydrure simple ou complexe de bore ou d'aluminium, par exemple, l'hydrure double de lithium et d'aluminium, l'hydrure d'aluminium, un complexe diborane/éther, ou le complexe diborane/sulfure de méthyle, ou tout autre moyen équivalent, au sein d'un solvant inerte tel que l'éther éthylique ou le tétrahydrofurane. La réduction peut être effectuée à température ambiante ou accélérée par chauffage jusqu'à la température de reflux du solvant.

Les exemples suivants illustrent l'invention sans toutefois en limiter la portée.

Les analyses élémentaires et les spectres IR et RMN confirment la structure des composés obtenue selon l'invention.

**Exemple 1**

**(Méthyl-3 benzoyl)-1 (1,4-benzodioxan-2 yl méthylaminométhyl)-4 pipéridine : composé n° 12 ; A = 0, B = 0, n = 1, $R_1$ = H, Y = CO, Ar = 3-Me.$C_6H_4$.**

Un mélange de 7 g de (méthyl-3 benzoyl)-1 (méthyl-4 benzènesulfonyloxyméthyl)-4 pipéridine et 3,2 g d'aminométhyl-2 1,4-benzodioxane est maintenu à 150°C sous agitation pendant deux heures. On laisse refroidir à température ambiante et on ajoute à la masse vitreuse du chloroforme et de l'ammoniaque à 10 %. On sépare la phase chloroformique qui est lavée à l'eau et séchée sur sulfate de sodium. Après filtration et évaporation du solvant, on purifie l'huile brute ainsi obtenue par chromatographie sur gel de silice en utilisant le chloroforme comme éluant. On obtient 4 g du composé 12 que l'on reprend dans 50 ml d'éthanol et traite avec 1,21 g d'acide maléique. On ajoute de l'éther éthylique à la solution et on obtient ainsi 3,82 g du maléate du composé 12 sous forme de cristaux blancs.
PF : 138-140°C.

**Exemple 2**

**(Méthyl-3 benzyl)-1 (1,4-benzodioxan-2 yl méthylaminométhyl)-4 pipéridine : composé n° 13 ; A = 0, B = 0, n = 1, $R_1$ = H, Y = $CH_2$, Ar = 3-Me.$C_6H_4$.**

On ajoute goutte à goutte une solution de 2,8 g de (méthyl-3 benzoyl)-1 (1,4-benzodioxan-2 yl méthylaminométhyl)-4 pipéridine obtenu selon la méthode décrite dans l'exemple 1 dans 15 ml de tétrahydrofurane à une suspension de 0,29 g de $LiAlH_4$ dans 10 ml de tétrahydrofurane en refroidissant à l'aide d'un bain de glace. On laisse remonter à température ambiante et après 16 heures d'agitation, le mélange réactionnel est hydrolysé par addition de soude 2N. On filtre, évapore le tétrahydrofurane sous pression réduite et on extrait la phare aqueuse par l'acétate d'éthyle. Les extraits sont lavés à l'eau salée, séchés sur sulfate de sodium et filtrés. L'huile obtenue par évaporation du solvant est purifiée par chromatographie sur gel de silice en utilisant un mélange chloroforme/méthanol (95/5) comme éluant. On obtient 1,1 g du composé 13 sous forme d'huile jaune pâle, que l'on reprend dans l'éthanol. On ajoute une solution éthanolique de 0,69 g d'acide maléique et on obtient, par l'addition d'acétate d'éthyle, 1,0 g de dimaléate du composé 13 sous forme de cristaux blancs.
PF : 195-197°C.

Le tableau 1 ci-après résume les principaux produits synthétisés qui illustrent l'invention sans toutefois en limiter la portée.

Tableau 1

| Composé n° | A | B | n | $R_1$ | Y | Ar | sel | PF(°C) |
|---|---|---|---|---|---|---|---|---|
| 1 | $CH_2$ | $CH_2$ | 0 | H | CO | 3-Me.$C_6H_4$ | Fumarate | 144-146 |
| 2 | $CH_2$ | $CH_2$ | 0 | H | $CH_2$ | 3-Me.$C_6H_4$ | Difumarate | 218-220 |
| 3 | O | $CH_2$ | 0 | H | CO | 3-Me.$C_6H_4$ | 1/2 Fumarate | 133-135 |
| 4 | S | $CH_2$ | 0 | H | $CH_2$ | 3-Me.$C_6H_4$ | Difumarate | 211-213 |
| 5 | $CH_2$ | $CH_2$ | 1 | H | CO | 3-Me.$C_6H_4$ | Maléate | 139-141 |
| 6 | $CH_2$ | $CH_2$ | 1 | H | $CH_2$ | 3-Me.$C_6H_4$ | Difumarate | 208-210 |
| 7 | O | $CH_2$ | 1 | H | CO | 3-Me.$C_6H_4$ | Fumarate | 141-143 |
| 8 | O | $CH_2$ | 1 | H | $CH_2$ | 3-Me.$C_6H_4$ | Difumarate | 203-205 |
| 9 | O | O | 1 | H | CO | $C_6H_5$ | Fumarate | 164-166 |
| 10 | O | O | 1 | H | $CH_2$ | $C_6H_5$ | Difumarate | 198-200 |
| 11 | O | O | 1 | H | $CH_2$ | 2-Me.$C_6H_4$ | Difumarate | 207-209 |
| 12 | O | O | 1 | H | CO | 3-Me.$C_6H_4$ | Maléate | 138-140 |
| 13 | O | O | 1 | H | $CH_2$ | 3-Me.$C_6H_4$ | Dimaléate | 195-197 |
| 14 | O | O | 1 | Me | CO | 3-Me.$C_6H_4$ | Fumarate | 138-140 |
| 15 | O | O | 1 | Me | $CH_2$ | 3-Me.$C_6H_4$ | Difumarate | 151-153 |
| 16 | O | O | 1 | H | CO | 4-Me.$C_6H_4$ | Fumarate | 129-131 |

**Tableau 1 (suite)**

| Composé n° | A | B | n | $R_1$ | Y | Ar | sel | PF(°C) |
|---|---|---|---|---|---|---|---|---|
| 17 | O | O | 1 | H | $CH_2$ | $4-Me.C_6H_4$ | Difumarate | 178-180 |
| 18 | O | O | 1 | H | CO | $2-F.C_6H_4$ | Fumarate | 128-130 |
| 19 | O | O | 1 | H | $CH_2$ | $2-F.C_6H_4$ | Difumarate | 189-191 |
| 20 | O | O | 1 | H | $CH_2$ | $3-MeO.C_6H_4$ | Difumarate | 203-205 |
| 21 | O | O | 1 | H | CO | $3-Cl.C_6H_4$ | Fumarate | 140-142 |
| 22 | O | O | 1 | H | $CH_2$ | $3-Cl.C_6H_4$ | Difumarate | 169-171 |
| 23 | O | O | 1 | H | CO | $3-CF_3.C_6H_4$ | Fumarate | 138-140 |
| 24 | O | O | 1 | H | $CH_2$ | $3-CF_3.C_6H_4$ | Difumarate | 214-216 |
| 25 | O | O | 1 | H | $CH_2$ | $3,4-(MeO)_2.C_6H_3$ | Difumar. | 204-206 |
| 26 | O | O | 0 | H | CO | $3-Me.C_6H_4$ | Fumarate | 137-139 |
| 27 | O | O | 1 | H | CO | $3,4-Cl_2.C_6H_3$ | Fumarate | 172-174 |
| 28 | O | S | 1 | H | CO | $3-Me.C_6H_4$ | Maléate | 114-116 |
| 29 | O | S | 1 | H | $CH_2$ | $3-Me.C_6H_4$ | Difumarate | 208-210 |
| 30 | O | O | 0 | H | $CH_2$ | $3-Me.C_6H_4$ | Difumarate | 195-197 |

Tableau 1 (suite)

| Composé n° | A | B | n | $R_1$ | Y | Ar | sel | PF(°C) |
|---|---|---|---|---|---|---|---|---|
| 31 | O | $CH_2$ | 1 | H | CO | $C_6H_5$ | Maléate | 151-153 |
| 32 | O | $CH_2$ | 1 | H | $CH_2$ | $C_6H_5$ | Dimaléate | 189-191 |
| 33 | O | $CH_2$ | 1 | H | CO | $3\text{-Cl.}C_6H_4$ | Maleate | 179-181 |
| 34 | O | $CH_2$ | 1 | H | $CH_2$ | $3\text{-Cl.}C_6H_4$ | Dimaleate | 190-192 |
| 35 | O | $CH_2$ | 1 | H | CO | $3\text{-MeO.}C_6H_4$ | Fumarate | 142-144 |
| 36 | O | $CH_2$ | 1 | H | $CH_2$ | $3\text{-MeO.}C_6H_4$ | Dimaleate | 196-198 |
| 37 | O | S | 1 | H | CO | $C_6H_5$ | Maleate | 147-149 |
| 38 | O | S | 1 | H | $CH_2$ | $C_6H_5$ | Difumarate | 206-208 |
| 39 | O | S | 1 | H | CO | $3\text{-Cl.}C_6H_4$ | Maleate | 127-129 |
| 40 | O | S | 1 | H | $CH_2$ | $3\text{-Cl.}C_6H_4$ | Difumarate | 196-198 |
| 41 | O | S | 1 | H | CO | $3\text{-MeO.}C_6H_4$ | Maleate | 108-110 |
| 42 | O | S | 1 | H | $CH_2$ | $3\text{-MeO.}C_6H_4$ | Dimaleate | 199-201 |

Les composés de l'invention ont été soumis à des essais pharmacologiques qui ont mis en évidence leur intérêt comme substances à activités thérapeutiques.

Ainsi, ils ont fait l'objet d'une étude portant sur leur affinité pour les récepteurs sérotoninergiques du type 5-$HT_{1A}$. Les études de binding 5-$HT_{1A}$ ont été réalisées selon la technique de Peroutka (cf. Peroutka S.J., J. Neurochem. 47, p.529-40, 1986). Des rats mâles Charles River sont assommés et décapités, le cerveau est prélevé et disséqué. Les différentes aires cérébrales obtenues sont utilisées ou conservées à moins 20°C. L'hippocampe est broyé au Polytron^R (20 secondes à 7) dans 20 volumes de tampon Tris-HCl 50 mM (pH 7,7 à 25°C) et centrifugé à 45000 g, 10 mn. Le culot est séparé, repris dans le même volume de tampon Tris et incubé 10 mn à 37°C puis recentrifugé 10 mn à 45000 g. Le culot est enfin repris dans 100 volumes de tampon Tris-HCl renfermant 10 μM de Pargyline, 4 mM $CaCl_2$ et 0,1 % d'acide ascorbique. Le mélange obtenu est homogénéisé au Dounce.

Le binding est réalisé à partir de 0,8 ml de la suspension membranaire précédente à laquelle on ajoute 0,1 ml de ligand $^3$H-8-OH-DPAT (à la concentration 1 nM) et soit 0,1 ml de tampon Tris (témoin), soit le composé de la présente invention. Après incubation 30 mn à 25°C, le mélange est filtré sur GF/B (Whatman), rincé avec

5 ml de tampon Tris-HCl glacé. Le résidu et le filtre sont enfin introduits dans une fiole renfermant 3 ml de liquide scintillant Instagel (Packard) et la radioactivité est mesurée au compteur Packard (Tri-Carbs). Les $CI_{50}$ sont déterminées graphiquement pour une concentration de ligand de 1 nM.

Le tableau 2 donne, à titre d'exemple, les $CI_{50}$ pour les récepteurs 5-$HT_{1A}$ pour certains dérivés de l'invention.

**Tableau 2**

| Composé n° | $CI_{50}$ nM |
|---|---|
| 3 | 7,5 |
| 5 | 12,5 |
| 7 | 3,8 |
| 9 | 3,0 |
| 17 | 4,4 |
| 22 | 2,1 |
| Buspirone | 23 |

Les résultats des essais montrent que les composés de formule générale 1 possèdent une grande affinité pour les récepteurs sérotoninergiques de type 5-$HT_{1A}$.

Les composés de l'invention peuvent donc être utiles pour le traitement de l'anxiété, la dépression, les troubles du sommeil, pour la régulation de la prise de nourriture, et pour le traitement des désordres vasculaires, cardiovasculaires et cérébrovasculaires tels que l'hypertension ou la migraine.

Les préparations pharmaceutiques contenant ces principes actifs peuvent être mises en forme pour l'administration par voie orale, rectale, parentérale ou locale, par exemple sous la forme de capsules, comprimés, granulés, gélules, solutés liquides, sirops ou suspensions buvables, et contenir les excipients appropriés.

Il est également possible d'y associer d'autres principes actifs pharmaceutiquement et thérapeutiquement acceptables.

## Revendications

**1)** Dérivés de l'aminométhyl-4 pipéridine correspondant à la formule générale 1

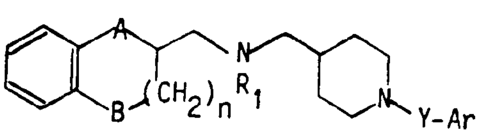

1

dans laquelle :

A et B représentent, indépendamment l'un de l'autre, un atome d'oxygène, un atome de soufre, ou un radical méthylène;

n peut prendre les valeurs de 0 ou 1 ;

$R_1$ représente un atome d'hydrogène ou un groupe alkyle en $C_1$-$C_4$ ;

Y représente un radical carbonyle ou un radical méthylène ;

Ar représente un groupe phényle substitué ou non par un ou plusieurs substituants choisis parmi un atome d'halogène, un groupe alkyle en $C_1$-$C_4$, un groupe alkoxy en $C_1$-$C_4$ ou un groupe trifluorométhyle.

ainsi que leurs sels avec des acides minéraux ou organiques thérapeutiquement acceptables, et pour les molécules renfermant un carbone asymétrique les mélanges racémiques, les énantiomères ou leurs mélanges en toutes proportions.

**2)** Composés de formule générale 1 selon la revendication 1 caractérisés par le fait qu'il sont choisis parmi :

(Méthyl-3 benzoyl)-1 (indan-2 yl méthylaminométhyl)-4 pipéridine
(Méthyl-3 benzyl)-1 (indan-2 yl méthylaminométhyl)-4 pipéridine
(Méthyl-3 benzoyl)-1 [(dihydro-2,3 benzofuran-2 yl) méthylaminométhyl]-4 pipéridine
(Méthyl-3 benzyl)-1 [(dihydro-2,3 benzothiophen-2 yl) méthylaminométhyl]-4 pipéridine
(Méthyl-3 benzoyl)-1 [(tétrahydro-1,2,3,4 naphtalen-2 yl) méthylaminométhyl]-4 pipéridine
(Méthyl-3 benzyl)-1 [(tétrahydro-1,2,3,4 naphtalen-2 yl) méthylaminométhyl]-4 pipéridine
(Méthyl-3 benzoyl)-1 [(dihydro-3,4 2[H].1-benzopyran-2 yl) méthylaminométhyl]-4 pipéridine
(Méthyl-3 benzyl)-1 [(dihydro-3,4 2[H].1-benzopyran-2 yl) méthylaminométhyl]-4 pipéridine
Benzoyl-1 (1,4-benzodioxan-2 yl méthylaminométhyl)-4 pipéridine
Benzyl-1 (1,4-benzodioxan-2 yl méthylaminométhyl)-4 pipéridine
(Méthyl-2 benzyl)-1 (1,4-benzodioxan-2 yl méthylaminométhyl)-4 pipéridine
(Méthyl-3 benzoyl)-1 (1,4-benzodioxan-2 yl méthylaminométhyl)-4 pipéridine
(Méthyl-3 benzyl)-1 (1,4-benzodioxan-2 yl méthylaminométhyl)-4 pipéridine
(Méthyl-3 benzoyl)-1 [N-méthyl N-(1,4-benzodioxan-2 yl méthyl) aminométhyl]-4 pipéridine
(Méthyl-3 benzyl)-1 [N-méthyl N-(1,4-benzodioxan-2 yl méthyl) aminométhyl]-4 pipéridine
(Méthyl-4 benzoyl)-1 (1,4-benzodioxan-2 yl méthylaminométhyl)-4 pipéridine
(Méthyl-4 benzyl)-1 (1,4-benzodioxan-2 yl méthylaminométhyl)-4 pipéridine
(Fluoro-2 benzoyl)-1 (1,4-benzodioxan-2 yl méthylaminométhyl)-4 pipéridine
(Fluoro-2 benzyl)-1 (1,4-benzodioxan-2 yl méthylaminométhyl)-4 pipéridine
(Méthoxy-3 benzyl)-1 (1,4-benzodioxan-2 yl méthylaminométhyl)-4 pipéridine
(Chloro-3 benzoyl)-1 (1,4-benzodioxan-2 yl méthylaminométhyl)-4 pipéridine
(Chloro-3 benzyl)-1 (1,4-benzodioxan-2 yl méthylaminométhyl)-4 pipéridine
(Trifluorométhyl-3 benzoyl)-1 (1,4-benzodioxan-2 yl méthylaminométhyl)-4 pipéridine
(Trifluorométhyl-3 benzyl)-1 (1,4-benzodioxan-2 yl méthylaminométhyl)-4 pipéridine
(Diméthoxy-3,4 benzyl)-1 (1,4-benzodioxan-2 yl méthylaminométhyl)-4 pipéridine
(Méthyl-3 benzoyl)-1 (1,3-benzodioxolan-2 yl méthylaminométhyl)-4 pipéridine
(Dichloro-3,4 benzoyl)-1 (1,4-benzodioxan-2 yl méthylaminométhyl)-4 pipéridine
(Méthyl-3 benzoyl)-1 (1,4-benzoxathian-2 yl méthylaminométhyl)-4 pipéridine
(Méthyl-3 benzyl)-1 (1,4-benzoxathian-2 yl méthylaminométhyl)-4 pipéridine
(Méthyl-3 benzyl)-1 (1,3-benzodioxolan-2 yl méthylaminométhyl)-4 pipéridine
Benzoyl-1[(dihydro-3,4 2 [H].1-benzopyran-2 yl) méthylaminométhyl]-4 pipéridine.
Benzyl-1[(dihydro-3,4 2 [H].1-benzopyran-2 yl) méthylaminométhyl]-4 pipéridine.
(Chloro-3 benzoyl)-1[(dihydro-3,4 2 [H].1-benzopyran-2 yl) méthylaminométhyl]-4 pipéridine.
(Chloro-3 benzyl)-1[(dihydro-3,4 2 [H].1-benzopyran-2 yl) méthylaminométhyl]-4 pipéridine.
(Méthoxy-3 benzoyl)-1[(dihydro-3,4 2 [H].1-benzopyran-2 yl) méthylaminométhyl]-4 pipéridine.
(Méthoxy-3 benzyl)-1[(dihydro-3,4 2 [H].1-benzopyran-2 yl) méthylaminométhyl]-4 pipéridine.
Benzoyl-1 (1,4-benzoxathian-2yl methylaminomethyl)-4 pipéridine.
Benzyl-1 (1,4-benzoxathian-2yl methylaminomethyl)-4 pipéridine.
(Chloro-3 benzoyl)-1(1,4-benzoxathian-2yl methylaminomethyl)-4 pipéridine.
(Chloro-3 benzyl)-1(1,4-benzoxathian-2yl methylaminomethyl)-4 pipéridine.
(Methoxy-3 benzoyl)-1(1,4-benzoxathian-2yl methylaminomethyl)-4 pipéridine.
(Methoxy-3 benzyl)-1(1,4-benzoxathian-2yl methylaminomethyl)-4 pipéridine.

**3)** Procédé de préparation de composés selon les revendications 1 et 2 caractérisé en ce que l'on fait réagir une amine de formule générale 2 avec un composé de formule générale 3 :

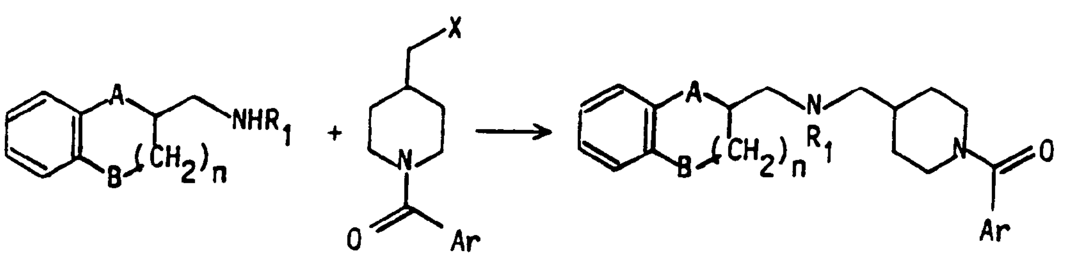

2 3 4

où

. A, B, n, $R_1$ et Ar sont définie comme ci-dessus.
. X représente un groupe nucléofuge tel que méthylsulfonyloxy, benzènesulfonyloxy ou p-toluènesulfonyloxy.
. les composés de formule générale 4 correspondent aux composés de formule générale 1 lorsque Y représente le radical carbonyle.

**4)** Procédé de préparation de composés selon la revendication 3 caractérisé en ce que la réaction d'une amine de formule générale 2 avec un composé de formule générale 3, s'effectue, soit en l'absence, soit en présence d'un solvant tel que le toluène, le xylène, le diméthylformamide ou l'acétonitrile de préférence à une température comprise entre 50 et 160°C, et éventuellement en présence d'une base organique telle qu'une amine tertaire, ou minérale, telle qu'un carbonate ou hydrogénocarbonate alcalin.

**5)** Procédé de préparation de composés selon les revendications 1 et 2, où Y représente le radical méthylène, caractérisé en ce que l'on réduit un composé de formule générale 4 selon le schéma :

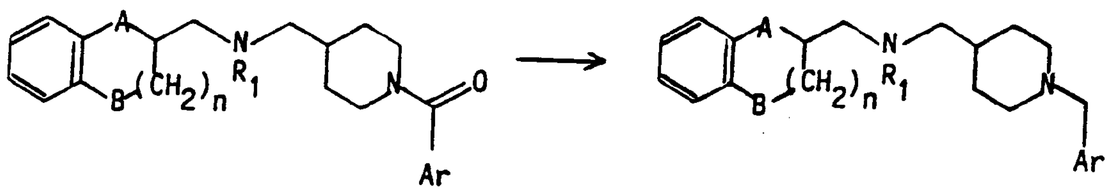

4

où A, B, n, $R_1$ et Ar sont définis comme ci-dessus.

**6)** Procédé de préparation de composés selon la revendication 5 caractérisé en ce que la réduction d'un composé de formule générale 4 s'effectue au moyen d'un hydrure simple ou complexe de bore ou d'aluminium, par exemple l'hydrure de lithium et d'aluminium, l'hydrure d'aluminium, un complexe diborane/éther, ou le complexe diborane/sulfure de méthyle, ou tout autre moyen équivalent, au sein d'un solvant inerte tel que l'éther éthylique ou le tétrahydrofurane à une température comprise entre la température ambiante et celle de reflux du solvant.

**7)** A titre de médicaments nouveaux utiles, par exemple, pour le traitement de l'anxiété, la dépression, les troubles du sommeil, pour la régulation de la prise de nourriture, et pour le traitement des désordres vasculaires, cardiovasculaires et cérébrovasculaires tels que l'hypertension ou la migraine, les composés définis selon l'une des revendications 1 et 2.

**8)** Composition pharmaceutique caractérisée en ce qu'elle contient à titre de principe actif au moins un composé selon l'une des revendications 1 et 2, associé à un véhicule pharmaceutique acceptable.

Office européen des brevets

# RAPPORT DE RECHERCHE EUROPEENNE

Numero de la demande

EP 92 40 2469
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 279 150 (ASTRA)<br>24 Août 1988<br>* le document en entier *<br>--- | 1-8 | C07D211/26<br>C07D405/12<br>C07D409/12<br>C07D411/12<br>A61K31/445 |
| A | EP-A-0 222 996 (CIBA-GEIGY)<br>27 Mai 1987<br>* le document en entier *<br>--- | 1-8 | |
| A | EP-A-0 170 213 (MERRELL-DOW)<br>5 Février 1986<br>* le document en entier *<br>--- | 1-8 | |
| A | JOURNAL OF MEDICINAL CHEMISTRY.<br>vol. 25, no. 2, 1982, WASHINGTON US<br>pages 136 - 141<br>DEMARINIS ET. AL. 'ALPHA-ADRENERGIC AGENTS'<br>* le document en entier *<br>--- | 1-8 | |
| A | JOURNAL OF MEDICINAL CHEMISTRY.<br>vol. 32, no. 8, 1989, WASHINGTON US<br>pages 1959 - 1962<br>CANNON ET. AL. '5-HT1A-RECEPTOR ANTAGONISM'<br>* le document en entier *<br>--- | 1-8 | **DOMAINES TECHNIQUES RECHERCHES (Int. Cl.5 )**<br>C07D<br>A61K |
| A | NATURE<br>vol. 305, 8 Septembre 1983,<br>pages 140 - 143<br>H. GOZLAN ET. AL. 'IDENTIFICATION OF PRESYNAPTIC SEROTONIN AUTORECEPTORS USING A NEW LIGAND: 3H-PAT'<br>* le document en entier *<br>---<br>-/-- | 1-8 | |

Le présent rapport a été établi pour toutes les revendications

| Lieu de la recherche | Date d'achèvement de la recherche | Examinateur |
|---|---|---|
| LA HAYE | 01 DECEMBRE 1992 | Bernd Kissler |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 92 40 2469
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | MOL. PHARM.<br>vol. 16, no. 3, 3 Novembre 1979, pages 687 - 699<br>S. J. PEROUTA, S. H. SNYDER 'MULTIPLE SEROTONIN RECEPTORS'<br>* le document en entier *<br>--- | 1-8 | |
| P,A | EP-A-0 452 204 (ADIR)<br>16 Octobre 1991<br>* le document en entier *<br>--- | 1-8 | |
| P,A | EP-A-0 457 686 (ADIR)<br>21 Novembre 1991<br>* le document en entier *<br>--- | 1-8 | |
| A | EP-A-0 445 026 (ADIR)<br>4 Septembre 1991<br>* le document en entier *<br>--- | 1-8 | |
| P,A | EP-A-0 447 292 (ADIR)<br>18 Septembre 1991<br>* le document en entier *<br>----- | 1-8 | DOMAINES TECHNIQUES RECHERCHES (Int. Cl.5 ) |

Le présent rapport a été établi pour toutes les revendications

| Lieu de la recherche | Date d'achèvement de la recherche | Examinateur |
|---|---|---|
| LA HAYE | 01 DECEMBRE 1992 | Bernd Kissler |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)